(19) Europäisches Patentamt / European Patent Office / Office européen des brevets

(11) **EP 3 950 035 A2**

(12) # EUROPEAN PATENT APPLICATION

(43) Date of publication:
**09.02.2022 Bulletin 2022/06**

(21) Application number: **21184271.1**

(22) Date of filing: **07.07.2021**

(51) International Patent Classification (IPC):
**A61M 25/00** (2006.01)   **A61M 25/01** (2006.01)
**A61M 25/06** (2006.01)

(52) Cooperative Patent Classification (CPC):
**A61M 25/0152; A61M 25/002;** A61M 25/0612;
A61M 25/0631

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.07.2020 JP 2020122666**

(71) Applicant: **FUJIFILM Corporation**
**Tokyo 106-8620 (JP)**

(72) Inventors:
• **DEJIMA, Takumi**
  **Kanagawa, 258-8538 (JP)**
• **OHKI, Tomohiro**
  **Kanagawa, 258-8538 (JP)**
• **NAITO, Satoshi**
  **Kanagawa, 258-8538 (JP)**

(74) Representative: **Klunker IP**
**Patentanwälte PartG mbB**
**Destouchesstraße 68**
**80796 München (DE)**

(54) ## CATHETER DEVICE AND CATHETER PACKAGE

(57)     Provided are a catheter device (10) and a catheter package (100) which are capable of preventing a bend from being formed in a catheter body (12).

The catheter body (12) includes a wire state discrimination portion (15) which discriminates a protruding state and a non-protruding state, in which the wire state discrimination portion includes a stopper (16) which allows a transition from the protruding state to the non-protruding state, and blocks a transition from the non-protruding state to the protruding state.

## FIG. 2

EP 3 950 035 A2

## Description

## BACKGROUND OF THE INVENTION

### 1. Field of the Invention

The present invention relates to a medical catheter device which is inserted into a body cavity and a catheter package.

### 2. Description of the Related Art

As a catheter device which is inserted into a body cavity, for example, a curved assembly of a medical flexible tube disclosed in JP1988-111833A (JP-S63-111833A) is known.

The curved assembly is configured by an outer cylinder member and an interpolation member inserted into the outer cylinder member, in which the interpolation member includes at least two or more bending portions to which a bend is imparted, and the outer cylinder member includes a restraining portion which restrains the bend of the bending portion. In this curved assembly, a curve control is performed by inserting the curved assembly into the body cavity, relatively moving back and forth the outer cylinder member and the interpolation member to change the positional relationship between the bending portion and the restraining portion.

## SUMMARY OF THE INVENTION

In the catheter device which is used by inserting the bent wire formed and bent in a curved shape into the catheter body, due to the relative position between the wire and the catheter body, in a case of delivery, a bend of the wire may be transferred to the catheter body, and a bend may be formed in the catheter body.

The present invention has been made in view of such circumstances, and an object of the present invention is to provide a catheter device and a catheter package which are capable of preventing a bend from being formed in a catheter body.

In order to achieve the object of the present invention, a catheter device according to an aspect of the present invention comprises a flexible catheter body which has a distal end opening and a proximal end opening, is inserted into a body cavity starting from the distal end opening, and includes a wire insertion passage provided therein; and a wire which has a higher rigidity than the catheter body, is inserted into the wire insertion passage, and includes a distal end portion and a proximal end portion, in which the distal end portion is formed in a curved shape, in which the catheter body includes a wire state discrimination portion which discriminates a protruding state in which the distal end portion of the wire protrudes from the distal end opening, and a non-protruding state in which the distal end portion of the wire does not protrude from the distal end opening, and the wire state discrimination portion includes an allowing and blocking member which allows a transition from the protruding state to the non-protruding state, and blocks a transition from the non-protruding state to the protruding state.

It is preferable that the catheter device according to the aspect of the present invention further comprise a moving object provided to be movable integrally with the wire, in which the allowing and blocking member includes a stopper which allows or restricts a movement of the moving object, and the stopper allows the movement of the moving object to enable the transition from the protruding state to the non-protruding state in a case in which the wire is moved in a direction from the distal end opening toward the proximal end opening, and restricts the movement of the moving object to disable the transition from the non-protruding state to the protruding state in a case in which the wire is moved in a direction from the proximal end opening toward the distal end opening.

In the catheter device according to the aspect of the present invention, it is preferable that the moving object be provided to be movable integrally with the wire in the wire insertion passage, and the stopper be provided in the wire insertion passage.

In the catheter device according to the aspect of the present invention, it is preferable that the stopper include a stepped portion provided in the wire insertion passage, and the moving object include a moving object side-climbing surface which abuts on the stepped portion and climbs over the stepped portion in a case in which the moving object is moved in a direction toward the proximal end opening, and a restricted surface which abuts on the stepped portion and of which a movement is restricted in a case in which the moving object is moved in a direction toward the distal end opening.

In the catheter device according to the aspect of the present invention, it is preferable that the stopper include a stepped portion provided in the wire insertion passage, and the stepped portion include a stopper side-climbing surface which abuts on the moving object and over which the moving object climbs in a case in which the moving object is moved in a direction toward the proximal end opening, and a restricting surface which abuts on the moving object and restricts the movement of the moving object in a case in which the moving object is moved in a direction toward the distal end opening.

In the catheter device according to the aspect of the present invention, it is preferable that at least one of the moving object or the stopper be made of an elastic member.

In the catheter device according to the aspect of the present invention, it is preferable that the catheter body include an outer circumferential surface, and a liquid supply port which supplies a liquid to the wire insertion passage be formed on the outer circumferential surface between the distal end opening and the proximal end opening, and a water stop valve which prevents the liquid from leaking to an outside from the proximal end opening be provided in the wire insertion passage between the liquid supply port and the proximal end opening.

In the catheter device according to the aspect of the present invention, it is preferable that the catheter body include a multi-lumen tube having the wire insertion passage and a liquid insertion passage, the catheter body include an outer circumferential surface, and a liquid supply port which supplies a liquid to the liquid insertion passage be formed on the outer circumferential surface on a proximal end opening side from the stopper, and in a longitudinal direction of the catheter body, a range from the liquid supply port to the stopper be configured as at least the multi-lumen tube.

In the catheter device according to the aspect of the present invention, it is preferable that in the longitudinal direction of the catheter body, the stopper be provided to be closer to the proximal end opening side than to a distal end opening side.

In the catheter device according to the aspect of the present invention, it is preferable that in the longitudinal direction of the catheter body, the stopper be provided to be closer to a distal end opening side than to the proximal end opening side.

In the catheter device according to the aspect of the present invention, it is preferable that in the longitudinal direction of the catheter body, the stopper be provided on a halfway side between the proximal end opening and the distal end opening.

In the catheter device according to the aspect of the present invention, it is preferable that the allowing and blocking member include an elastic valve member provided in the wire insertion passage, an insertion hole through which the wire is inserted be provided in the elastic valve member, and the elastic valve member close the insertion hole in a case in which the wire is not disposed in the insertion hole.

It is preferable that the catheter device according to the aspect of the present invention further comprise a wire operating portion which operates the wire in a wire axis direction and a direction around a wire axis, in which the wire operating portion is connected to the proximal end portion of the wire extended from the proximal end opening to an outside.

It is preferable that the catheter device according to the aspect of the present invention further comprise a fixing member which temporarily fixes a position of the wire relative to the catheter body, in which the fixing member is provided in the proximal end portion of the wire extended from the proximal end opening to an outside.

In order to achieve the object of the present invention, a catheter package according to another aspect of the present invention comprises a catheter device, and a package body which accommodates the catheter device, in which the catheter device includes a flexible catheter body which has a distal end opening and a proximal end opening, is inserted into a body cavity starting from the distal end opening, and includes a wire insertion passage provided therein, and a wire which has a higher rigidity than the catheter body, is inserted into the wire insertion passage, and includes a distal end portion and a proximal end portion, in which the distal end portion is formed in a curved shape, and the catheter device is accommodated in the package body in a protruding state in which the distal end portion of the wire protrudes from the distal end opening.

According to the present invention, it is possible to prevent a bend from being formed in the catheter body.

## BRIEF DESCRIPTION OF THE DRAWINGS

Fig. 1 is an overall perspective view of a catheter device according to a first embodiment.
Fig. 2 is an enlarged cross sectional view of a main portion including a partially broken portion of the catheter device shown in Fig. 1.
Fig. 3 is an enlarged cross sectional view of a first restricting portion and the vicinity thereof.
Fig. 4 is an operation explanatory view of the catheter device shown in Fig. 1.
Fig. 5 is an overall view of a catheter package according to the embodiment.
Fig. 6 is a flowchart showing an example of a treatment example in a case in which the catheter device shown in Fig. 1 is used for DB-ERCP.
Fig. 7 is an enlarged cross sectional view of a main portion of a catheter device according to a second embodiment.
Fig. 8 is a schematic configuration view of a catheter device according to a third embodiment.
Fig. 9 is a schematic configuration view of a catheter device according to a fourth embodiment.
Fig. 10 is a schematic configuration view of a catheter device according to a fifth embodiment.
Fig. 11 is a schematic configuration view of a catheter device according to a sixth embodiment.
Fig. 12 is an explanatory view showing a state in which a distal end of a slide wire abuts on a valve member shown in Fig. 11.

## DESCRIPTION OF THE PREFERRED EMBODIMENTS

Hereinafter, a catheter device and a catheter package according to an embodiment of the present invention will be described with reference to the accompanying drawings.

First Embodiment

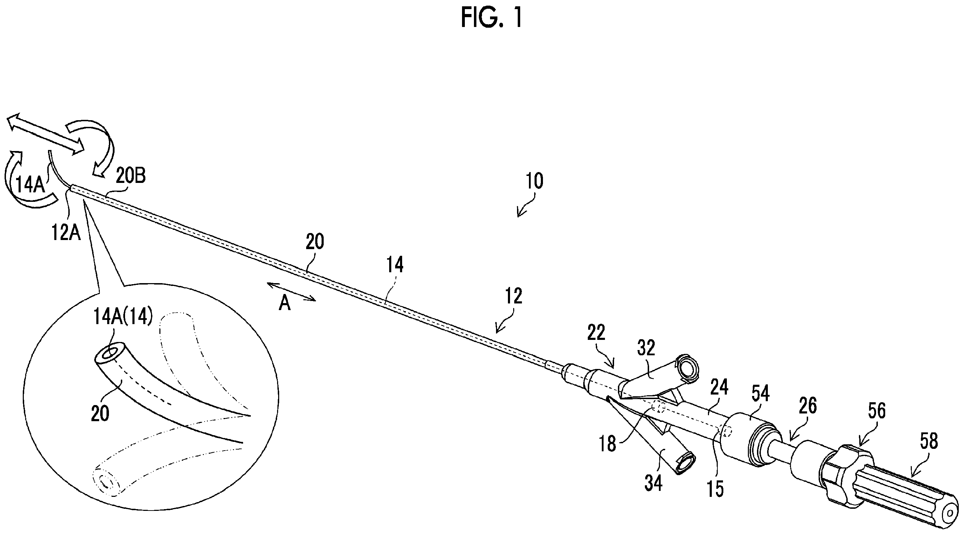

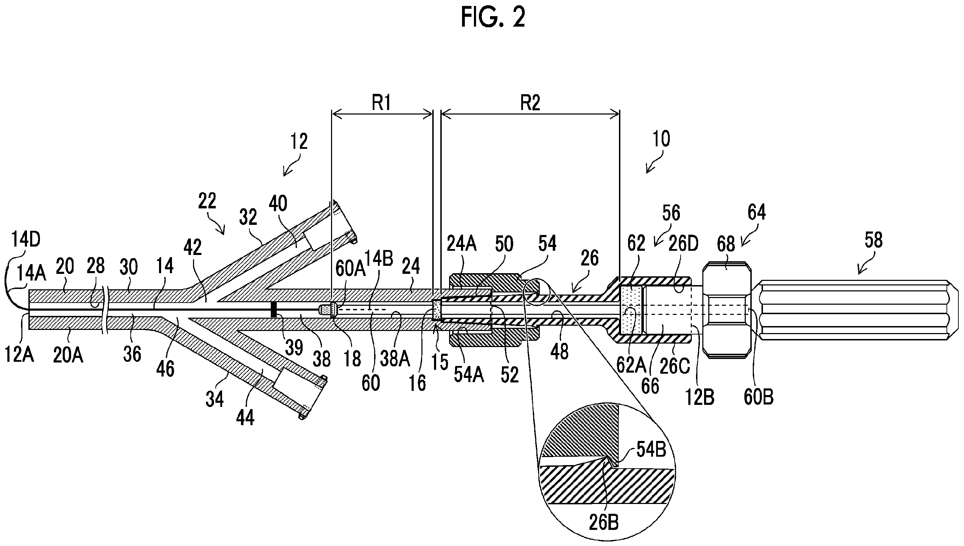

Fig. 1 is an overall perspective view of a catheter device 10 according to a first embodiment. Fig. 2 is an enlarged cross sectional view of a main portion including a partially broken portion of the catheter device 10 shown in Fig. 1.

As shown in Figs. 1 and 2, the catheter device 10 according to the first embodiment comprises a catheter body 12 and a slide wire 14.

The catheter body 12 according to the present embodiment includes a long tube 20 which is inserted into a body cavity in a case of clinical use, a three-way tube 22 which is connected to a proximal end portion of the tube 20, and a connecting tube 26 which is connected to a proximal end portion of a first tube 24 which configures the three-way tube 22, in a longitudinal direction A shown in Fig. 1. Further, the catheter body 12 has a configuration which a distal end opening 12A is provided in a distal end portion of the tube 20, and a proximal end opening 12B (see Fig. 2) is provided in a proximal end portion of the connecting tube 26.

As shown in Fig. 2, the tube 20 is configured by a single lumen tube in which one passage 28 which communicates the distal end portion with the proximal end portion is provided. The passage 28 functions as one passage which configures a wire insertion passage of the slide wire 14, and the distal end portion of the passage 28 is communicated with the distal end opening 12A. Further, the tube 20 is formed of a flexible material which is inserted into the curved body cavity. The material of the tube 20 is not particularly limited as long as it has flexibility that enables movement along the body cavity, and examples thereof include a synthetic resin or the like used for a known catheter.

The three-way tube 22 includes a main tube 30, and a first tube 24, a second tube 32, and a third tube 34 which are respectively connected to a proximal end side of the main tube 30. The distal end portion of the main tube 30 is connected to the proximal end portion of the tube 20, the main tube 30 and the first tube 24 are disposed along the same axis, and the second tube 32 and the third tube 34 are disposed to face each other with the first tube 24 interposed therebetween.

In the three-way tube 22 according to the present embodiment, a passage 36 of the main tube 30 and a passage 38 of the first tube 24 function together with the passage 28 as one passage which configures the wire insertion passage. Further, a passage 40 of the second tube 32 and the passage 36 are communicated with each other via a liquid supply port 42 formed on an outer circumferential surface of the main tube 30 and function as an injection passage of a liquid such as a contrast medium. Further, a passage 44 of the third tube 34 and the passage 36 are communicated with each other via a guide wire insertion port 46 formed on the outer circumferential surface of the main tube 30 and function as an insertion passage of a guide wire (not shown) inserted into, for example, an inside of a bile duct (not shown). The liquid or the guide wire is ejected or protrudes from the distal end opening 12A via the passage 28 in a case of clinical use.

Further, a water stop valve 39 is provided in the passage 38. The water stop valve 39 is formed in a disk shape by, for example, an elastic member such as rubber, and the outer peripheral portion thereof is attached to an inner wall 38A of the passage 38, and an insertion hole (not shown) into which the slide wire 14 is inserted is formed in the center portion thereof. The water stop valve 39 is provided on the proximal end opening 12B side from the liquid supply port 42, and has a function of stopping the contrast medium flowed back into the passage 38 among the contrast media supplied from the liquid supply port 42. This makes it possible to prevent the contrast medium from leaking from the proximal end opening 12B.

A passage 48 which communicates the distal end portion with the proximal end portion is provided in the connecting tube 26. The proximal end portion of the passage 48 is communicated with the proximal end opening 12B. The passage 48 is disposed on the same axis with the passage 38 in a case in which the distal end portion 50 of the connecting tube 26 is connected to the proximal end portion of the first tube 24 as will be described below. As a result, the passage 48 functions as one passage which configures the wire insertion passage together with the passage 28, the passage 36, and the passage 38, and in the catheter body 12 according to the present embodiment, the four passages 28, 36, 38, and 48 which communicate with each other configure the wire insertion passage.

The catheter body 12 includes a wire state discrimination portion 15 in the passage 38. The wire state discrimination portion 15 will be described below.

The distal end portion 50 of the connecting tube 26 is formed in a truncated cone shape that the outer diameter is reduced toward the distal end opening 12A. By inserting the distal end portion 50 into the proximal end opening 52 of the first tube 24, the distal end portion 50 is connected to the proximal end portion of the first tube 24 as described above. Further, in order to strengthen the connection described above, the catheter body 12 according to the present embodiment has a nut 54.

The nut 54 is formed in a tubular shape and is attached externally on the connecting tube 26, a female screw 54A is formed on an inner circumferential surface on the distal end side, and a flange 54B is formed on the inner circumferential surface on the proximal end side. Further, a male screw 24A into which the female screw 54A is screwed is formed on the outer circumferential surface of the first tube 24 on the proximal end side, and a flange 26B on which the flange 54B abuts is formed on the outer circumferential surface of the connecting tube 26. With the nut 54, in a case in which the female screw 54A is screwed into the male screw 24A, the flange 54B abuts on the flange 26B and presses the flange 26B in the direction toward the distal end opening 12A. As a result, the outer circumferential surface of the distal end portion 50 is pressed on the inner circumferential surface of the proximal end opening 52, and thus the connection described above can be strengthened. A braking portion 56 and a handle 58 are provided at the proximal end portion of the connecting tube 26, and the description of the braking portion 56 and the handle 58 will be made below.

Hereinafter, the slide wire 14 will be described.

The slide wire 14 is an example of the wire according to the embodiment of the present invention and has a higher rigidity than the tube 20. The slide wire 14 is inserted into the passages 28, 36, 38, and 48, which are the wire insertion passages, as will be described below. In this case, the shape of the tube 20 is determined by the shape of the slide wire 14 inserted into the passage 28, and is elastically deformed along the shape of the slide wire 14. For example, the slide wire 14 is formed of a metal wire made of stainless steel or a nickel-titanium alloy.

The slide wire 14 includes a distal end portion 14A and a proximal end portion 14B (see Fig. 2), and in particularly, the distal end portion 14A is formed in a curved shape in order to elastically deform the distal end portion 20A of the tube 20 in a curved shape in a case of clinical use. As shown in Figs. 1 and 2, the distal end portion 14A can protrude from the distal end opening 12A of the tube 20 to the outside.

As shown in Fig. 2, the proximal end portion 14B of the slide wire 14 is inserted into a thin metal guide tube 60, which has a rigidity and is inserted into the passages 38 and 48, and is fixed to the distal end portion 60A of the guide tube 60. The guide tube 60 is extended from the proximal end opening 12B to the outside, and the proximal end portion 60B of the extended guide tube 60 is connected to the handle 58 via the braking portion 56. The guide tube 60 configures a part of the slide wire 14 as a whole.

For example, the slide wire 14 configured as described above is inserted, starting from the proximal end portion 60B of the guide tube 60, into the passages 28, 36, 38, and 48 in this order from the distal end opening 12A of the tube 20. Therefore, the slide wire 14 is inserted into the wire insertion passage of the catheter body 12 together with the guide tube 60. A moving object 18 is provided in the slide wire 14. The moving object 18 will be described below.

Hereinafter, the braking portion 56 and the handle 58 will be described.

As shown in Fig. 2, the braking portion 56 is an example of a fixing member, and includes a brake pad 62 and a fastening member 64. The brake pad 62 is formed in a tubular shape by an elastic member such as silicon rubber, and is attached to the inside of a cylindrical portion 26C formed in the proximal portion of the connecting tube 26. Further, the brake pad 62 has an insertion hole 62A formed therein, and the guide tube 60 is inserted and disposed in the insertion hole 62A so as to be rotatable, and capable of being pushed and pulled. The guide tube 60 is also inserted into the fastening member 64, and the proximal end portion 60B thereof is connected to the handle 58 as described above.

The fastening member 64 is formed in a tubular shape as a whole, and a male screw portion 66 is provided in the distal end portion and a knob portion 68 is provided in the proximal end portion. The male screw portion 66 is screwed into a female screw 26D formed on the inner circumferential surface of the cylindrical portion 26C. In this state, in a case in which the male screw portion 66 is screwed into the female screw 26D by rotating the knob portion 68, the brake pad 62 is interposed and compressed between the male screw portion 66 and the cylindrical portion 26C. Then, the diameter of the insertion hole 62A is reduced and the intimate attachment force of the brake pad 62 to the guide tube 60 is increased, so that the rotating operation and the pushing and pulling operation of the guide tube 60 are restricted by the brake pad 62. As a result, the position of the slide wire 14 relative to the catheter body 12 is temporarily fixed.

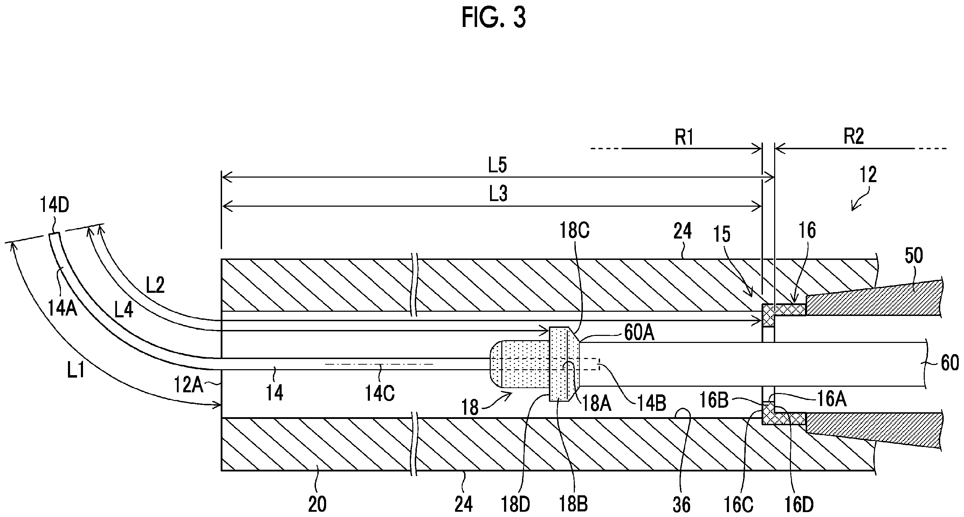

On the other hand, the handle 58 is an example of a wire operating portion, and the proximal end portion of the guide tube 60 is connected to the distal end portion thereof. Therefore, the slide wire 14 can be rotated in the direction around an axis 14C (see Fig. 3) via the guide tube 60 by the rotating operation of the handle 58, and the slide wire 14 can be moved in a direction of the axis 14C via the guide tube 60 by the pushing and pulling operation of the handle 58.

Hereinafter, the wire state discrimination portion 15 will be described.

As shown in Fig. 2, the catheter device 10 according to the first embodiment comprises the wire state discrimination portion 15 which discriminates a protruding state in which the distal end portion 14A of the slide wire 14 protrudes from the distal end opening 12A, and a non-protruding state in which the distal end portion 14A of the slide wire 14 does not protrude from the distal end opening 12A. The wire state discrimination portion 15 includes a stopper 16 that is provided in the passage 38 of the catheter body 12 and functions as an allowing and blocking member which allows a transition from the protruding state to the non-protruding state, and blocks a transition from the non-protruding state to the protruding state.

The stopper 16 has a function of discriminating a region in which the slide wire 14 is movable into a protruding region R1 in which the protruding state is enabled, and a non-protruding region R2 in which the non-protruding state is enabled, by allowing or restricting the movement of the moving object 18 to be described below. The protruding region R1 and the non-protruding region R2 will be described below.

Further, the stopper 16 is disposed on the inner wall 38A of the passage 38 as an example. Specifically, the stopper 16 is provided in the distal end of the distal end portion 50 of the connecting tube 26, and is disposed in the inner wall 38A of the passage 38 by connecting the distal end portion 50 to the proximal end opening 52 of the first tube 24 as shown in Fig. 2. The disposition form of the stopper 16 is not limited to the above form, and the stopper 16 may be provided directly on the inner wall 38A.

Fig. 3 is an enlarged cross sectional view of the stopper 16 and the vicinity thereof.

As shown in Fig. 3, as an example, the stopper 16 is formed in a tubular shape, and an insertion hole 16A into which the slide wire 14 including the guide tube 60 is inserted is formed in the center portion thereof. Further, the inner peripheral portion of the stopper 16, which includes the inner circumferential surface of the insertion hole 16A, is configured as a stepped portion 16B. The surface on the distal end side of the stepped portion 16B is formed as a vertical surface 16C which is vertical to the axis 14C of the slide wire 14, and the surface on the proximal end side of the stepped portion 16B is formed as a vertical surface 16D which is vertical to the axis 14C of the slide wire 14. The stopper 16 is made of an elastic member such as rubber, as an example.

Hereinafter, the moving object 18 will be described.

As shown in Fig. 3, the moving object 18 is disposed in the passage 36 and fixed to the distal end portion 60A of the guide tube 60 which is moved together with the slide wire 14. As a result, the moving object 18 is provided to be movable integrally with the slide wire 14. The fixed form of the moving object 18 is not limited to the above form, and the moving object 18 may be directly fixed to the slide wire 14.

As an example, the moving object 18 is formed in a tubular shape, and an insertion hole 18A into which the proximal end portion 14B of the slide wire 14 is inserted is formed in the center portion thereof. Further, the outer peripheral portion of the moving object 18, which includes the outer circumferential surface, is configured as a stepped portion 18B. The surface on the proximal end side of the stepped portion 18B is formed as a truncated cone surface 18C of which the outer diameter is reduced toward the proximal end opening 12B (see Fig. 2), and the surface on the distal end side of the stepped portion 18B is formed as a vertical surface 18D which is vertical to the axis 14C of the slide wire 14.

The truncated cone surface 18C functions as a moving object side-climbing surface which can climb over the stepped portion 16B of the stopper 16 in a case in which the slide wire 14 is moved in a direction from the distal end opening 12A toward the proximal end opening 12B (see Fig. 2) in the protruding region R1 to be described below.

Further, the vertical surface 18D functions as a restricted surface which abuts on the stepped portion 16B of the stopper 16 and restricts the movement of the moving object 18 in a case in which the slide wire 14 is moved in a direction from the proximal end opening 12B toward the distal end opening 12A in the non-protruding region R2 to be described below.

The moving object 18 configured as described above may be made of an elastic member as in the stopper 16, but in a case in which the stopper 16 is made of an elastic member, for example, it may be made of a hard member such as plastic. That is, at least one of the moving object 18 or the stopper 16 may be made of an elastic member.

Next, an example for setting the protruding region R1 and the non-protruding region R2 will be described with reference to Fig. 3.

About Protruding Region R1

As described above, the protruding region R1 is a region in the moving region of the slide wire 14, which enables the protruding state. The protruding region R1 is set, for example, as follows.

That is, as shown in Fig. 3, a total length of the curved shaped distal end portion 14A of the slide wire 14 is L1, and this total length L1 protrudes from the distal end opening 12A to the outside. In that case, a length of the slide wire 14 from a distal end 14D of the slide wire 14 to the vertical surface 16C of the stopper 16 is L2. Then, a length of the catheter body 12 from the distal end opening 12A to the vertical surface 16C is L3. In this case, L1, L2, and L3 are set by satisfying the relationship of Expression (1) below.

$$(L2 - L1) > L3 \ (1)$$

About Non-protruding Region R2

As described above, the non-protruding region R2 is a region in the moving region of the slide wire 14, which enables the non-protruding state. The non-protruding region R2 is set, for example, as follows.

That is, as shown in Fig. 3, a length from the distal end 14D of the slide wire 14 to the vertical surface 18D of the moving object 18 is L4. Then, a length of the catheter body 12 from the distal end opening 12A to the vertical surface 16D of the stopper 16 is L5. In this case, L4 and L5 are set by satisfying the relationship of Expression (2) below.

$$L4 \leq L5 \ (2)$$

That is, the protruding region R1 and the non-protruding region R2 are set by providing the stopper 16 and the moving object 18 at positions satisfying Expression (1) and (2) above, respectively. In this case, with the stopper 16 as a boundary, the protruding region R1 is set in the direction toward the distal end opening 12A, and the non-protruding region R2 is set in the direction toward the proximal end opening 12B.

Further, the stopper 16 has a function of allowing the movement of the moving object 18 to enable the transition from the protruding state to the non-protruding state in a case in which the slide wire 14 is moved in the direction from the distal end opening 12A toward the proximal end opening 12B, and restricting the movement of the moving object 18 to disable the transition from the non-protruding state to the protruding state in a case in which the slide wire 14 is moved in the direction from the proximal end opening 12B toward the distal end opening 12A.

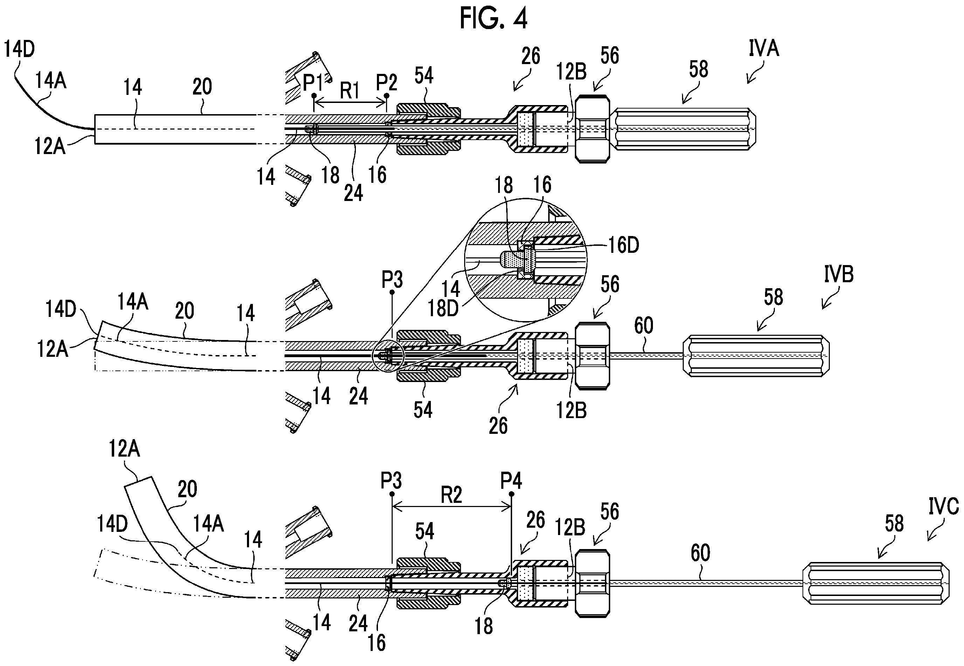

Hereinafter, a specific example of the function described above of the stopper 16 will be described with reference to the operation explanatory view of the catheter body 12 shown in Fig. 4.

IVA of Fig. 4 shows a state of the catheter device 10 in a case in which the slide wire 14 is most moved in the direction toward the distal end opening 12A by the handle 58. In this case, the moving object 18 is positioned at a most distal end position P1 of the protruding region R1, and the distal end portion 14A of the slide wire 14 is in the protruding state in which the total length thereof protrudes from the distal end opening 12A. In this protruding state, in a case in which the slide wire 14 is moved in the direction toward the proximal end opening 12B by the handle 58, the moving object 18 is moved from the position P1 toward a most proximal end position P2 (position of the vertical surface 16C of the stopper 16) of the protruding region R1, and the distal end portion 14A of the slide wire 14 is accommodated in the passage 28 (see Fig. 2) starting from the distal end opening 12A of the tube 20. Then, in a case in which the moving object 18 reaches the position P2, the distal end portion 14A of the slide wire 14 is accommodated in the passage 28 and leaves, for example, a part of the distal end portion 14A (for example, the distal end 14D). In this case, the distal end portion 20A of the tube 20 is elastically deformed in a curved shape along the curved shape of the distal end portion 14A.

Next, in a case in which the slide wire 14 is further moved in the direction toward the proximal end opening 12B by the handle 58 from the position in which the moving object 18 reaches the position P2, due to the force, the truncated cone surface 18C of the moving object 18 shown in Fig. 3 advances while expanding the insertion hole 16A. As a result, the stepped portion 18B climbs over the stepped portion 16B, and the moving object 18 is positioned at the most distal end position P3 of the non-protruding region R2 from the position P2 (see IVB of Fig. 4). That is, in a case in which the slide wire 14 is moved in the direction from the distal end opening 12A toward the proximal end opening 12B, the stopper 16 allows the movement of the moving object 18 and enables the transition from the protruding state to the non-protruding state.

In this case, as shown in IVB of Fig. 4, the total length of the distal end portion 14A of the slide wire 14 is accommodated in the passage 28. Then, in this state, in a case in which the slide wire 14 is attempted to be moved in the direction toward the distal end opening 12A by the handle 58, the vertical surface 18D of the moving object 18 abuts on the vertical surface 16D of the stopper 16, and the movement of the moving object 18 is restricted. That is, in a case in which the slide wire 14 is moved in the direction from the proximal end opening 12B toward the distal end opening 12A, the stopper 16 restricts the movement of the moving object 18 and disables the transition from the non-protruding state to the protruding state. The specific function of the stopper 16 is described above.

On the other hand, in the form of IVB of Fig. 4, in a case in which the slide wire 14 is further moved in the direction toward the proximal end opening 12B by the handle 58, the moving object 18 is moved toward the proximal end opening 12B, and the distal end portion 14A of the slide wire 14 is further accommodated toward the proximal end side of the passage 28. Then, as shown in IVC of Fig. 4, in a case in which the moving object 18 abuts on the brake pad 62, that is, in a case in which the moving object 18 reaches the most proximal end position P4 of the non-protruding region R2, the movement of the slide wire 14 by the handle 58 in the direction toward the proximal end opening 12B is restricted.

As described above, in the catheter device 10 according to the present embodiment, a region between the position P1 and the position P2 is set as the protruding region R1, and a region between the distal end position P3 and the proximal end position P4 is set as the non-protruding region R2.

Next, an example of a form of the catheter device 10 in a case of delivery and a case of clinical use configured as described above will be described.

Form in Case of Delivery

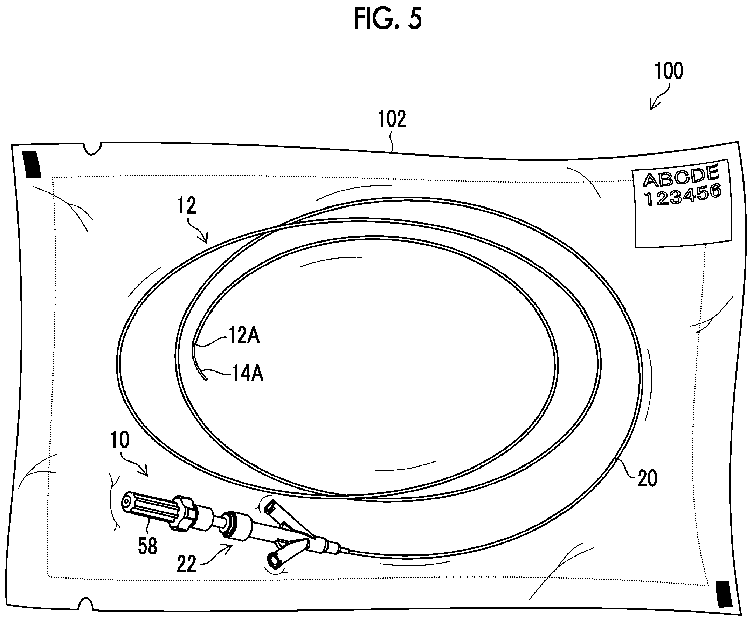

Fig. 5 is an overall view of a catheter package 100 according to the embodiment of the present invention, and shows a form of the catheter device 10 in a case of delivery.

As shown in Fig. 5, the catheter package 100 according to the present embodiment includes the catheter device 10 and a package body 102, and the catheter device 10 is accommodated in the package body 102.

In the package body 102, an accommodating portion which is blocked from the external space is formed. Furthermore, the package body 102 is made of a transparent material.

The catheter device 10 is accommodated in the package body 102 in the protruding state in which the total length of the curved shaped distal end portion 14A protrudes from the distal end opening 12A of the tube 20. This form is the form of the catheter device 10 in a case of delivery.

As described above, in the catheter device 10 according to the present embodiment, the relative position between the catheter body 12 and the slide wire 14 in a case of delivery can be defined as a position that enables the protruding state, so that a bend of the distal end portion 14A is prevented from being transferred to the tube 20 in a case of delivery. Therefore, it is possible to prevent a bend from being formed in the catheter body 12.

Form in Case of Clinical Use

In a case of clinical use, the package body 102 of Fig. 5 is opened, and the catheter device 10 is extracted from the package body 102. In this case, since the distal end portion 14A is in the protruding state, the slide wire 14 is moved in the direction toward the proximal end opening 12B by the handle 58, and the protruding state is transitioned to the non-protruding state. This form is the form of the catheter device 10 in a case of clinical use.

As described above, in the catheter device 10 according to the present embodiment, the relative position between the catheter body 12 and the slide wire 14 in a case of clinical use can be defined as a position that enables the non-protruding state. This makes it possible to prevent the distal end portion 14A of the slide wire 14 from protruding toward the inside of the body cavity in a case of clinical use.

As described above, with the catheter device 10 according to the first embodiment, the catheter body 12 includes the wire state discrimination portion 15 which discriminates the protruding state and the non-protruding state, and the wire state discrimination portion 15 includes the stopper 16 as an allowing and blocking member which allows the transition from the protruding state to the non-protruding state, and blocks the transition from the non-protruding state to the protruding state, so that the suitable relative position between the catheter body 12 and the slide wire 14 in a case of delivery and clinical use can be defined.

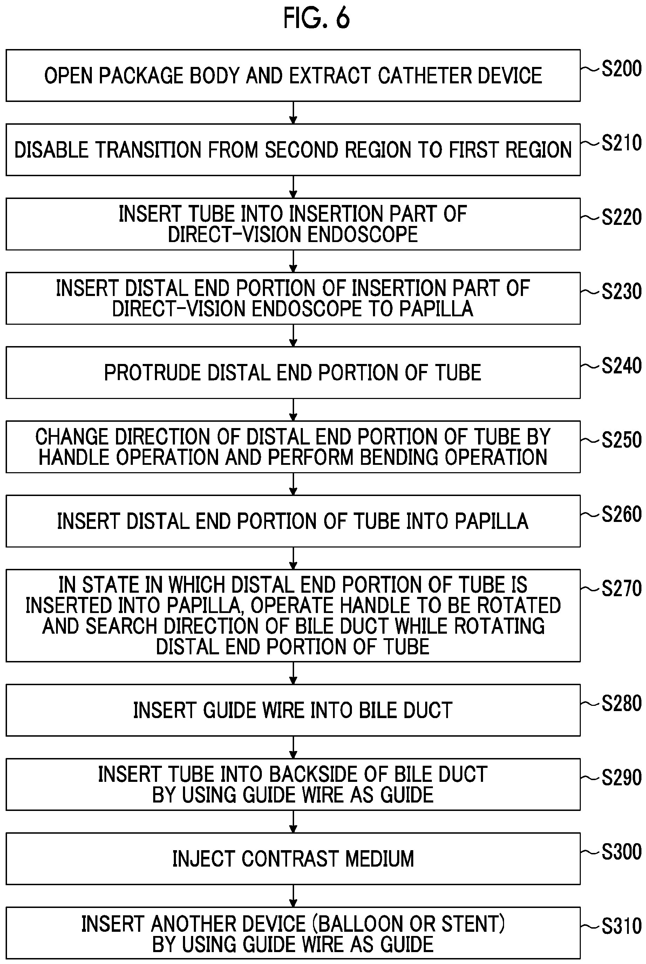

Next, an example of a treatment example in a case in which the catheter device 10 according to the present embodiment is used for double balloon endoscopic retrograde cholangiopancreatography (DB-ERCP) will be described with reference to a flowchart shown in Fig. 6.

At first, in step (S) 200, the package body 102 is opened, and the catheter device 10 is extracted from the package body 102.

In this case, since the distal end portion 14A of the slide wire 14 is in the protruding state, in S210, the slide wire 14 is moved in the direction toward the proximal end opening 12B by the handle 58, and the protruding state is transitioned to the non-protruding state. An operator who operates the handle 58 can confirm that the protruding state is transitioned to the non-protruding state with the feeling of operation of a case in which the stepped portion 18B shown in Fig. 3 climbs over the stepped portion 16B.

Next, in S220, a double-balloon endoscope, which is a direct-vision endoscope, is used, and the tube 20 is inserted, starting from the distal end opening 12A of the tube 20, into the insertion part of the double-balloon endoscope from the forceps port of the double-balloon endoscope. In this case, since the distal end portion 14A of the slide wire 14 does not protrude from the distal end opening 12A, the tube 20 can be smoothly inserted into the insertion part.

Next, in S230, the distal end portion of the insertion part of the double-balloon endoscope is inserted from the mouth to the vicinity of the papilla of the duodenum while appropriately expanding and contracting the balloon. This insertion operation is performed while observing a body cavity image sent from an imaging unit provided at the distal end portion of the insertion part on a monitor.

Next, in S240, the distal end portion 20A of the tube 20 protrudes from the distal end portion of the insertion part. In this case, the distal end portion 20A is elastically deformed in a curved shape along the shape of the curved shaped distal end portion 14A of the slide wire 14 accommodated in the distal end portion 20A.

Next, in S250, the handle 58 of the catheter device 10 is operated such that the distal end portion 20A is directed toward the papilla while observing the image of the papilla and the distal end portion 20A of the tube 20 displayed on the monitor. That is, in a case in which the handle 58 is rotated, the slide wire 14 rotates around the axis 14C, so that the curved distal end portion 20A of the tube 20 can be rotated around the axis 14C. Further, in a case in which the handle 58 is pulled or pushed in, the slide wire 14 is moved along the axis 14C, so that the curved angle of the distal end portion 20A of the tube 20 can be changed. Such an operation is appropriately performed to direct the distal end portion 20A toward the papilla.

Next, in S260, the distal end portion 20A of the tube 20 is inserted into the papilla.

Next, in S270, in a state in which the distal end portion 20A of the tube 20 is inserted into the papilla, the handle 58 is operated to be rotated, the direction of the bile duct is searched while rotating the distal end portion 20A of the tube 20, and the direction of the distal end portion 20A is aligned with the direction of the bile duct. Thereafter, the slide wire 14 is fixed to be inoperative by the braking portion 56 shown in Fig. 2, and the direction of the distal end portion 20A is fixed.

Next, in S280, the guide wire is inserted from the passage 44 of the three-way tube 22 shown in Fig. 2, and the distal end portion of the guide wire protrudes from the distal end opening 12A of the tube 20 and inserted into the bile duct.

Next, in S290, the tube 20 is inserted into the back side of the bile duct by using the guide wire as a guide, if necessary.

Next, in S300, if necessary, the contrast medium is sent from the passage 40 of the three-way tube 22 shown in Fig. 2, and the contrast medium is injected into the bile duct from the distal end opening 12A of the tube 20.

Next, in S310, if necessary, another device such as a balloon or a stent is inserted into the bile duct by using the guide wire as a guide to treat the affected area.

The above is an example of a treatment example by using the catheter device 10 according to the present embodiment.

Here, the difference of a treatment method between the DB-ERCP described above and the ERCP of a normal case will be briefly described.

In the ERCP, the duodenoscopy is inserted from the mouth to the duodenum and the distal end portion of the tube is derived from the distal end portion of the duodenoscopy. Next, the treatment tool elevator provided in the distal end portion of the duodenoscopy is operated to align the direction of the distal end portion of the tube with the direction of the papilla, and then the distal end portion of the tube 20 is inserted into the bile duct via the papilla (hereinafter referred to as "cannulation").

On the other hand, in the DB-ERCP, since the duodenoscopy cannot be inserted into the papilla, a direct-vision endoscope such as a double-balloon endoscope is used instead of the duodenoscopy, but the direct-vision endoscope does not comprise the treatment tool elevator. In addition, the direction of the papilla varies, especially in the postoperative intestine. Due to such circumstances, in the DB-ERCP, it is very difficult to align the distal end portion of the tube with the direction of the papilla, and it may not be possible to easily perform cannulation.

Therefore, in a case in which the catheter device 10 according to the present embodiment is used for the DB-ERCP, in the catheter device 10 according to the present embodiment, the direction of the distal end portion 20A of the tube 20 can be freely adjusted by the rotating operation and the pushing and pulling operation of the handle 58, and thus the direction of the distal end portion 20A of the tube 20 can be easily aligned with the direction of the papilla. As a result, it is possible to easily perform cannulation.

Some embodiments of the catheter device according to the present invention will be described below.

Second Embodiment

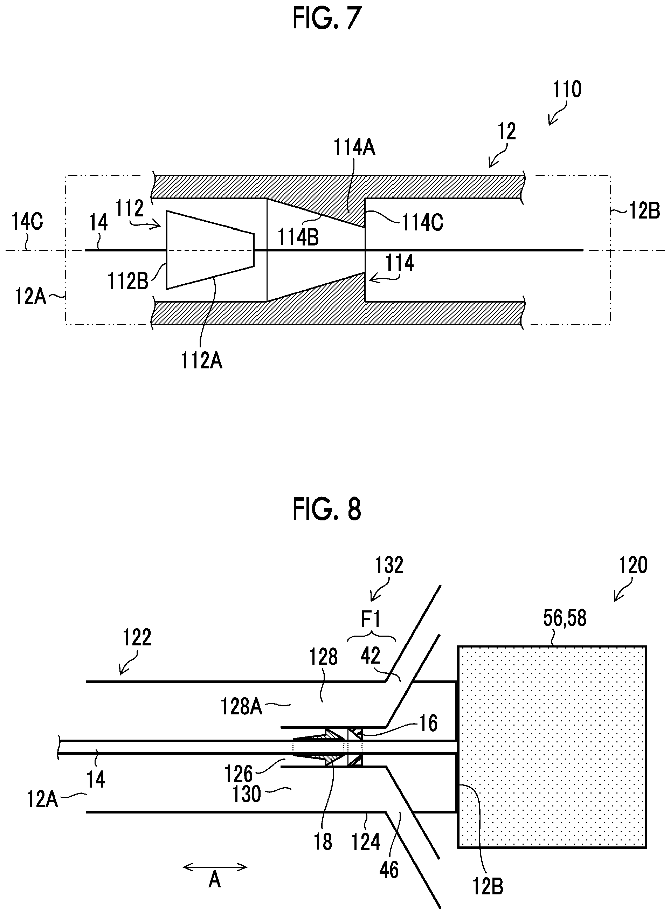

Fig. 7 is an enlarged cross sectional view of a main portion of a catheter device 110 according to a second embodiment, and shows a moving object 112 and a stopper 114. The difference between the catheter device 110 according to the second embodiment and the catheter device 10 according to the first embodiment shown in Figs. 1 to 5 is the shape of the moving object 112 and the stopper 114 shown in Fig. 7, and other portions are the same, the description thereof will be omitted.

In the moving object 112 shown in Fig. 7, the slide wire 14 is inserted and disposed therein, and the moving object 112 is fixed to the slide wire 14. The moving object 112 is formed in a truncated cone shape that the outer diameter is reduced in a direction toward the proximal end opening 12B, and the outer circumferential surface thereof is formed as a truncated cone surface 112A. Further, the surface on the distal end side of the moving object 112 is formed as a vertical surface 112B which is vertical to the axis 14C.

Further, the stopper 114 includes a stepped portion 114A. The stepped portion 114A includes a truncated cone surface 114B which abuts on the truncated cone surface 112A of the moving object 112 and over which the moving object 112 climbs, in a case in which the moving object 112 is moved in the direction toward the proximal end opening 12B. Further, the stepped portion 114A includes a vertical surface 114C which abuts on the vertical surface 112B of the moving object 112 and restricts the movement of the moving object 112, in a case in which the moving object 112 is moved in the direction toward the distal end opening 12A. Here, the truncated cone surface 114B functions as a stopper side-climbing surface, and the vertical surface 114C functions as a restricting surface.

With the catheter device 110 according to the second embodiment, in a case in which the slide wire 14 is moved in the direction from the distal end opening 12A toward the proximal end opening 12B, the stopper 114 allows the movement of the moving object 112 and enables the transition from the protruding state to the non-protruding state. Also, in a case in which the slide wire 14 is moved in the direction from the proximal end opening 12B toward the distal end opening 12A, the stopper 114 restricts the movement of the moving object 112 and disables the transition from the non-protruding state to the protruding state. As a result, the catheter device 110 according to the second embodiment can prevent a bend from being formed in the catheter body 12 as in the catheter device 10 according to the first embodiment, and can define the suitable relative position between the catheter body and the wire in a case of delivery and clinical use.

Third Embodiment

Fig. 8 is a schematic cross sectional view of the catheter device 120 according to a third embodiment, and shows a tube 122 including a multi-lumen tube 124.

As shown in Fig. 8, the multi-lumen tube 124 includes a wire insertion passage 126, a liquid passage 128 which is communicated with the liquid supply port 42, and a guide wire insertion passage 130 which is communicated with the guide wire insertion port 46.

Further, a catheter body 132 shown in Fig. 8 includes the stopper 16 to be closer to the proximal end opening 12B side than to the distal end opening 12A (see Fig. 2) side in the longitudinal direction A, and the liquid supply port 42 is formed on the proximal end opening 12B side from the stopper 16. A range F1 from the liquid supply port 42 to the stopper 16 is configured by the multi-lumen tube 124 described above.

With the catheter device 120 configured in this way, the contrast medium supplied from the liquid supply port 42 to the liquid passage 128 is smoothly flowed from the distal end opening 128A of the liquid passage 128 into the tube 122 configured by the single lumen tube. As described above, by configuring the range F1 from the liquid supply port 42 to the stopper 16 by the multi-lumen tube 124, the backflow of the contrast medium to the proximal end opening 12B side can be prevented without using the water stop valve 39 for backflow prevention shown in Fig. 2.

The range of the multi-lumen tube 124 in the tube 122 is not limited to the range F1, at least the range F1 need only be configured by the multi-lumen tube 124, and thus the range beyond the range F1 may be configured by the multi-lumen tube 124. For example, the total length of the tube 122 may be configured by the multi-lumen tube 124.

Further, in the multi-lumen tube 124 shown in Fig. 8, the liquid passage 128 and the guide wire insertion passage 130 are respectively independently configured, but a multi-lumen tube may be adopted in which the liquid passage 128 and the guide wire insertion passage 130 are configured as one passage.

Fourth Embodiment

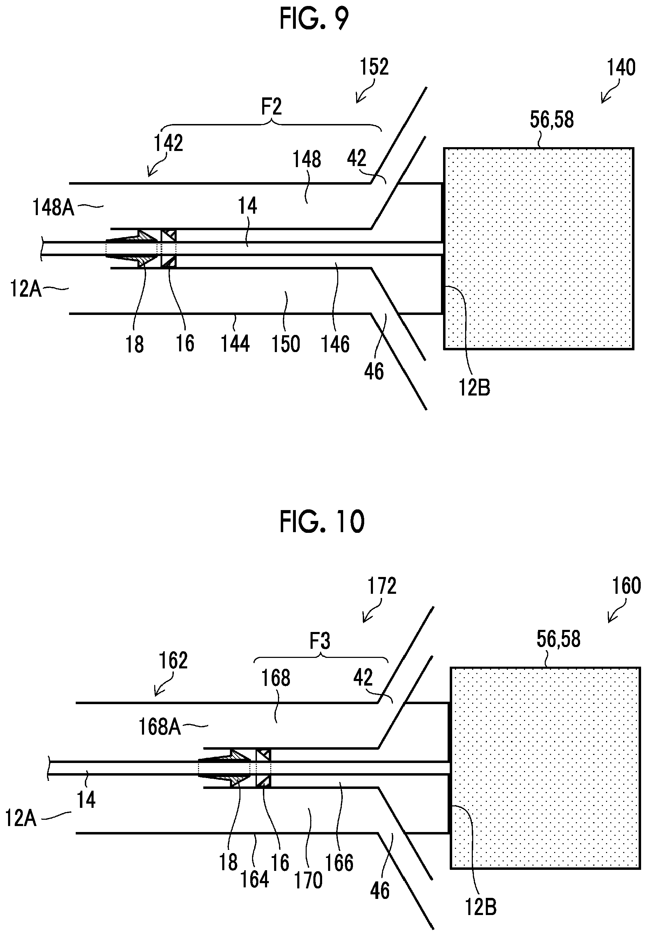

Fig. 9 is a schematic cross sectional view of the catheter device 140 according to a fourth embodiment, and shows a tube 142 including a multi-lumen tube 144. The difference between the catheter device 140 according to the fourth embodiment and the catheter device 120 according to the third embodiment shown in Fig. 8 is that the stopper 16 shown in Fig. 9 is disposed to be closer to the distal end opening 12A side than to the proximal end opening 12B side.

As shown in Fig. 9, the multi-lumen tube 144 includes a wire insertion passage 146, a liquid passage 148 which is communicated with the liquid supply port 42, and a guide wire insertion passage 150 which is communicated with the guide wire insertion port 46.

Further, in a catheter body 152 shown in Fig. 9, the liquid supply port 42 is formed on the proximal end opening 12B side from the stopper 16. A range F2 from the liquid supply port 42 to the stopper 16 is configured by the multi-lumen tube 144.

With the catheter device 140 configured in this way, the contrast medium supplied from the liquid supply port 42 to the liquid passage 148 is smoothly flowed from the distal end opening 148A of the liquid passage 148 into the tube 142 configured by the single lumen tube. As described above, by configuring the range F2 from the liquid supply port 42 to the stopper 16 by the multi-lumen tube 144, the backflow of the contrast medium to the proximal end opening 12B side can be prevented without using the water stop valve 39 for backflow prevention shown in Fig. 2.

The range of the multi-lumen tube 144 in the tube 142 is not limited to the range F2, at least the range F2 need only be configured by the multi-lumen tube 144, and thus the range beyond the range F2 may be configured by the multi-lumen tube 144. For example, the total length of the tube 142 may be configured by the multi-lumen tube 144.

Fifth Embodiment

Fig. 10 is a schematic cross sectional view of the catheter device 160 according to a fifth embodiment, and shows a tube 162 including a multi-lumen tube 164. The difference between the catheter device 160 according to the fifth embodiment and the catheter device 120 according to the third embodiment shown in Fig. 8 is that the stopper 16 shown in Fig. 10 is disposed on a halfway side between the proximal end opening 12B and the distal end opening 12A.

As shown in Fig. 10, the multi-lumen tube 164 includes a wire insertion passage 166, a liquid passage 168 which is communicated with the liquid supply port 42, and a guide wire insertion passage 170 which is communicated with the guide wire insertion port 46.

Further, in a catheter body 172 shown in Fig. 10, the liquid supply port 42 is formed on the proximal end opening 12B side from the stopper 16. A range F3 from the liquid supply port 42 to the stopper 16 is configured by the multi-lumen tube 164 described above.

With the catheter device 160 configured in this way, the contrast medium supplied from the liquid supply port 42 to the liquid passage 168 is smoothly flowed from the distal end opening 168A of the liquid passage 168 into the tube 162 configured by the single lumen tube. As described above, by configuring the range F3 from the liquid supply port 42 to the stopper 16 by the multi-lumen tube 164, the backflow of the contrast medium to the proximal end opening 12B side can be prevented without using the water stop valve 39 for backflow prevention shown in Fig. 2.

The range of the multi-lumen tube 164 in the tube 162 is not limited to the range F3, at least the range F3 need only be configured by the multi-lumen tube 164, and thus the range beyond the range F3 may be configured by the multi-lumen tube 164. For example, the total length of the tube 162 may be configured by the multi-lumen tube 164.

Further, in the multi-lumen tube 164 shown in Fig. 10, the liquid passage 168 and the guide wire insertion passage 170 are respectively independently configured, but the present invention is not limited to this, and a multi-lumen tube may be adopted in which the liquid passage 168 and the guide wire insertion passage 170 are configured as one passage.

In the first to fifth embodiments described above, the catheter device in which the stopper and the moving object are respectively provided in the wire insertion passage is described as an example, but the catheter device according to the embodiment of the present invention is not limited to these embodiments. For example, a configuration may be adopted in which an adapter which comprises the stopper, and configures a part of the catheter body is attached to the outside of the catheter body, the wire is arranged on the adapter, and the stopper in the adapter allows or restricts the movement of the moving object. That is, the stopper need only be provided in the catheter body.

Further, in the first embodiment shown in Figs. 1 and 2, the catheter body 12 configured by the tube 20, the three-way tube 22, and the connecting tube 26 is described as an example, but the present invention is not limited to this, and for example, the catheter body may be configured by only the tube 20. In this case, the tube 20 need only be provided with the stopper 16.

Sixth Embodiment

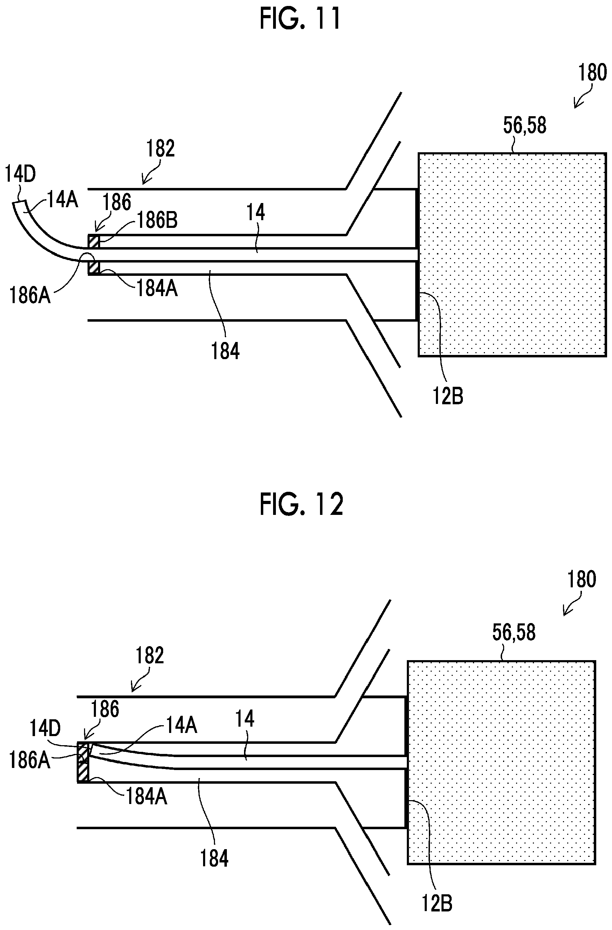

Fig. 11 is a schematic cross sectional view of a catheter device 180 according to a sixth embodiment.

In the catheter device 180 shown in Fig. 11, a tube 182 is configured by the multi-lumen tube. A valve member 186 which functions as an allowing and blocking member is attached to a distal end opening 184A of a wire insertion passage 184 in the multi-lumen tube.

The valve member 186 is an elastic valve member made of an elastic member such as rubber, and is attached to the distal end opening 184A by fixing the outer peripheral portion thereof to the inner wall of the distal end opening 184A. Further, an insertion hole 186A into which the slide wire 14 is inserted is formed in the center portion of the valve member 186.

According to the sixth embodiment, as shown in Fig. 11, in a case of delivery, the distal end portion 14A of the slide wire 14 protrudes from the insertion hole 186A of the valve member 186. As a result, it is possible to prevent a bend of the distal end portion 14A from being transferred to the tube 182, so that it is possible to prevent a bend from being formed in the catheter body. Further, as shown in Fig. 12, in a case of clinical use, the distal end portion 14A is accommodated in the wire insertion passage 184. As a result, the insertion hole 186A is closed, and it is possible to prevent the distal end portion 14A from protruding toward the inside of the body cavity.

In this case, it is preferable that the diameter of the wire insertion passage 184 be made larger than the diameter of the slide wire 14. Then, in a case in which the distal end portion 14A is accommodated in the wire insertion passage 184, the relative position between the distal end 14D and the insertion hole 186A shifts in the radial direction of the valve member 186, so that the distal end 14D can be blocked from being inserted again into the insertion hole 186A. As a result, the protrusion described above can be surely prevented.

The embodiments of the present invention have been described above, but the present invention is not limited to the above examples, and it is noted that various modifications or changes may be made without departing from the gist of the present invention. For example, the package body 102 shown in Fig. 5 can accommodate the catheter devices 110, 120, 140, 160, and 180 according to the second to sixth embodiments.

Hereinafter, an example of a catheter device different from that of the present invention will be described.

In the present invention, the transition from the non-protruding state to the protruding state is blocked by the allowing and blocking member, but in a case of clinical use, in a case in which the distal end portion 14A may protrude from the distal end opening 12A, the transition from the non-protruding state to the protruding state may be allowed.

In this case, for example, in the stopper 114 shown in Fig. 7 need only have a configuration in which the shape of the vertical surface 114C is formed into the truncated cone surface, and the movement of the moving object 112 is allowed in a case in which the slide wire 14 is moved in the direction from the proximal end opening 12B toward the distal end opening 12A.

Explanation of References

10: catheter device
12: catheter body
12A: distal end opening
12B: proximal end opening
14: slide wire
14A: distal end portion
14B: proximal end portion
14C: axis
14D: distal end
15: wire state discrimination portion
16: stopper
16A: insertion hole
16B: stepped portion
16C: vertical surface
16D: vertical surface
18: moving object
18A: insertion hole
18B: stepped portion
18C: truncated cone surface
18D: vertical surface
20: tube
20A: distal end portion
22: three-way tube
24: first tube
24A: male screw
26: connecting tube
26B: flange
26C: cylindrical portion
26D: female screw
28: passage
30: main tube
32: second tube
34: third tube
36: passage
38: passage
38A: inner wall

39: water stop valve
40: passage
42: liquid supply port
44: passage
46: guide wire insertion port
48: passage
50: distal end portion
52: proximal end opening
54: nut
54A: female screw
54B: flange
56: braking portion
58: handle
60: guide tube
60A: distal end portion
60B: proximal end portion
62: brake pad
62A: insertion hole
64: fastening member
66: male screw portion
68: knob portion
100: catheter package
102: package body
110: catheter device
112: moving object
112A: truncated cone surface
112B: vertical surface
114: stopper
114A: stepped portion
114B: truncated cone surface
114C: vertical surface
120: catheter device
122: tube
124: multi-lumen tube
126: wire insertion passage
128: liquid passage
130: guide wire insertion passage
132: catheter body
140: catheter device
142: tube
144: multi-lumen tube
146: wire insertion passage
148: liquid passage
150: guide wire insertion passage
152: catheter body
160: catheter device
162: tube
164: multi-lumen tube
166: wire insertion passage
168: liquid passage
170: guide wire insertion passage
172: catheter body
180: catheter device
182: tube
184A: distal end opening
186: valve member
186A: insertion hole
R1: protruding region

R2: non-protruding region

**Claims**

1. A catheter device comprising:

   a flexible catheter body which has a distal end opening and a proximal end opening, is inserted into a body cavity starting from the distal end opening, and includes a wire insertion passage provided therein; and
   a wire which has a higher rigidity than the catheter body, is inserted into the wire insertion passage, and includes a distal end portion and a proximal end portion, in which the distal end portion is formed in a curved shape,
   wherein the catheter body includes a wire state discrimination portion which discriminates a protruding state in which the distal end portion of the wire protrudes from the distal end opening, and a non-protruding state in which the distal end portion of the wire does not protrude from the distal end opening, and
   the wire state discrimination portion includes an allowing and blocking member which allows a transition from the protruding state to the non-protruding state, and blocks a transition from the non-protruding state to the protruding state.

2. The catheter device according to claim 1, further comprising:

   a moving object is provided to be movable integrally with the wire,
   wherein the allowing and blocking member includes a stopper which allows or restricts a movement of the moving object, and
   the stopper allows the movement of the moving object to enable the transition from the protruding state to the non-protruding state in a case in which the wire is moved in a direction from the distal end opening toward the proximal end opening, and restricts the movement of the moving object to disable the transition from the non-protruding state to the protruding state in a case in which the wire is moved in a direction from the proximal end opening toward the distal end opening.

3. The catheter device according to claim 2,

   wherein the moving object is provided to be movable integrally with the wire in the wire insertion passage, and
   the stopper is provided in the wire insertion passage.

**4.** The catheter device according to claim 3,

wherein the stopper includes a stepped portion provided in the wire insertion passage, and the moving object includes a moving object side-climbing surface which abuts on the stepped portion and climbs over the stepped portion in a case in which the moving object is moved in a direction toward the proximal end opening, and a restricted surface which abuts on the stepped portion and of which a movement is restricted in a case in which the moving object is moved in a direction toward the distal end opening.

**5.** The catheter device according to claim 3,

wherein the stopper includes a stepped portion provided in the wire insertion passage, and the stepped portion includes a stopper side-climbing surface which abuts on the moving object and over which the moving object climbs in a case in which the moving object is moved in a direction toward the proximal end opening, and a restricting surface which abuts on the moving object and restricts the movement of the moving object in a case in which the moving object is moved in a direction toward the distal end opening.

**6.** The catheter device according to any one of claims 2 to 5,
wherein at least one of the moving object or the stopper is made of an elastic member.

**7.** The catheter device according to any one of claims 1 to 6,

wherein the catheter body includes an outer circumferential surface, and a liquid supply port which supplies a liquid to the wire insertion passage is formed on the outer circumferential surface between the distal end opening and the proximal end opening, and a water stop valve which prevents the liquid from leaking to an outside from the proximal end opening is provided in the wire insertion passage between the liquid supply port and the proximal end opening.

**8.** The catheter device according to any one of claims 2 to 6,

wherein the catheter body includes a multi-lumen tube having the wire insertion passage and a liquid insertion passage, the catheter body includes an outer circumferential surface, and a liquid supply port which supplies a liquid to the liquid insertion passage is formed on the outer circumferential surface on a proximal end opening side from the stopper, and in a longitudinal direction of the catheter body, a range from the liquid supply port to the stopper is configured as at least the multi-lumen tube.

**9.** The catheter device according to claim 8, wherein in the longitudinal direction of the catheter body, the stopper is provided to be closer to the proximal end opening side than to a distal end opening side.

**10.** The catheter device according to claim 8, wherein in the longitudinal direction of the catheter body, the stopper is provided to be closer to a distal end opening side than to the proximal end opening side.

**11.** The catheter device according to claim 8, wherein in the longitudinal direction of the catheter body, the stopper is provided on a halfway side between the proximal end opening and the distal end opening.

**12.** The catheter device according to claim 1,

wherein the allowing and blocking member includes an elastic valve member provided in the wire insertion passage, an insertion hole through which the wire is inserted is provided in the elastic valve member, and the elastic valve member closes the insertion hole in a case in which the wire is not disposed in the insertion hole.

**13.** The catheter device according to any one of claims 1 to 12, further comprising a wire operating portion which operates the wire in a wire axis direction and a direction around a wire axis, wherein the wire operating portion is connected to the proximal end portion of the wire extended from the proximal end opening to an outside.

**14.** The catheter device according to any one of claims 1 to 13, further comprising a fixing member which temporarily fixes a position of the wire relative to the catheter body, wherein the fixing member is provided in the proximal end portion of the wire extended from the proximal end opening to an outside.

**15.** A catheter package comprising:

a catheter device; and a package body which accommodates the catheter device,

**EP 3 950 035 A2**

wherein the catheter device includes a flexible catheter body which has a distal end opening and a proximal end opening, is inserted into a body cavity starting from the distal end opening, and includes a wire insertion passage provided therein, and a wire which has a higher rigidity than the catheter body, is inserted into the wire insertion passage, and includes a distal end portion and a proximal end portion, in which the distal end portion is formed in a curved shape, and

the catheter device is accommodated in the package body in a protruding state in which the distal end portion of the wire protrudes from the distal end opening.

FIG. 1

EP 3 950 035 A2

FIG. 2

FIG. 3

FIG. 4

# FIG. 5

## FIG. 6

| | |
|---|---|
| OPEN PACKAGE BODY AND EXTRACT CATHETER DEVICE | ~S200 |
| DISABLE TRANSITION FROM SECOND REGION TO FIRST REGION | ~S210 |
| INSERT TUBE INTO INSERTION PART OF DIRECT-VISION ENDOSCOPE | ~S220 |
| INSERT DISTAL END PORTION OF INSERTION PART OF DIRECT-VISION ENDOSCOPE TO PAPILLA | ~S230 |
| PROTRUDE DISTAL END PORTION OF TUBE | ~S240 |
| CHANGE DIRECTION OF DISTAL END PORTION OF TUBE BY HANDLE OPERATION AND PERFORM BENDING OPERATION | ~S250 |
| INSERT DISTAL END PORTION OF TUBE INTO PAPILLA | ~S260 |
| IN STATE IN WHICH DISTAL END PORTION OF TUBE IS INSERTED INTO PAPILLA, OPERATE HANDLE TO BE ROTATED AND SEARCH DIRECTION OF BILE DUCT WHILE ROTATING DISTAL END PORTION OF TUBE | ~S270 |
| INSERT GUIDE WIRE INTO BILE DUCT | ~S280 |
| INSERT TUBE INTO BACKSIDE OF BILE DUCT BY USING GUIDE WIRE AS GUIDE | ~S290 |
| INJECT CONTRAST MEDIUM | ~S300 |
| INSERT ANOTHER DEVICE (BALLOON OR STENT) BY USING GUIDE WIRE AS GUIDE | ~S310 |

# FIG. 7

# FIG. 8

## FIG. 9

## FIG. 10

# FIG. 11

# FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 63111833 A

- JP S63111833 A